**Europäisches Patentamt**

**European Patent Office**

**Office européen des brevets**

(19)

(11) Publication number : **0 446 858 B1**

# EUROPEAN PATENT SPECIFICATION

(12)

(45) Date of publication of patent specification :
27.09.95 Bulletin 95/39

(51) Int. Cl.$^6$ : **G01N 21/27, G01N 33/53**

(21) Application number : **91103738.0**

(22) Date of filing : **12.03.91**

(54) Calibrating method of enzyme immuno assay system.

(30) Priority : **13.03.90 JP 62191/90**

(43) Date of publication of application :
**18.09.91 Bulletin 91/38**

(45) Publication of the grant of the patent :
**27.09.95 Bulletin 95/39**

(84) Designated Contracting States :
**AT BE DE ES FR GB IT NL**

(56) References cited :
**EP-A- 0 083 761
DD-A- 242 872
DE-A- 1 911 576
US-A- 3 435 237
US-A- 4 401 387
REVIEW OF SCIENTIFIC INSTRUMENTS. vol. 49, no. 8, August 1978, NEW YORK US pages 1134 - 1136 ; M. MICHAUD ET AL. : "Optical absorption using a system response corrector"
PATENT ABSTRACTS OF JAPAN vol. 8, no. 254 (P-315)(1691) 21 November 1984, & JP-A-59 125043**

(73) Proprietor : **Sankyo Company Limited
5-1 Nihonbashi Honcho 3-chome
Chuo-ku
Tokyo (JP)**

Proprietor : **HORIBA, LTD.
2 Miyanohigashi-machi
Kissyoin
Minami-ku Kyoto (JP)**

(72) Inventor : **Saito, Yukio
19-3, Tomiokanishi 6-chome,
Kanazawa-ku
Yokohama-shi, Kanagawa-ken (JP)**
Inventor : **Sekiya, Koichi
13-8, Inage 3-chome
Ciba-shi, Ciba-ken (JP)**
Inventor : **Sato, Yoshihiro
448-20, Kuden-cho,
Sakae-ku
Yokohama-shi, Kanagawa-ken (JP)**
Inventor : **Kohno, Takeshi
4-44-7-207, Miyanosaka
Hirakata-shi, Osaka (JP)**
Inventor : **Takahasi, Hiroaki, 11-202, Ogurusu
Kita Danti
6 Ogurusu,
Minami Goto-cho
Fusimi-ku, Kyoto (JP)**
Inventor : **Yamamoto, Kenji
45-1, Minamitatsumi-cho
Nishikyo-ku, Kyoto (JP)**

(74) Representative : **TER MEER - MÜLLER - STEINMEISTER & PARTNER
Mauerkircherstrasse 45
D-81679 München (DE)**

EP 0 446 858 B1

## Description

## BACKGROUND OF THE INVENTION

### Field of the Invention

The present invention relates to a calibrating method as claimed in the preamble of claim 1 and, in particular, to a calibrating method in an enzyme immuno assay system in which an intensity of a chemical luminescence generated in a photometric cell is detected by means of two optical detectors different in sensitivity of measurement.

### Description of the Prior Art

An enzyme immuno assay system measures substances to be tested, such as carcino embryonic antigen (CEA), ferritin (FER), $\alpha$-fetoprotein (AFP) and thyroxine binding globulin (TBG), in a blood according to the enzyme immuno method. This measuring method is outlined as follows:

A sample (serum) to be measured is put in an antibody immobilized tube with an antibody immobilized therein and then a suitable enzyme conjugated antibody reagent is added to carry out an immunological reaction. Subsequently, a substrate solution is added to carry out an enzyme reaction, whereby generating hydrogen peroxide, and a part of the resulting reaction liquid containing hydrogen peroxide is put in a photometric cell together with a luminescent reagent (luminol) to detect an intensity of a chemical luminescence generated in said photometric cell, whereby measuring said substances to be tested.

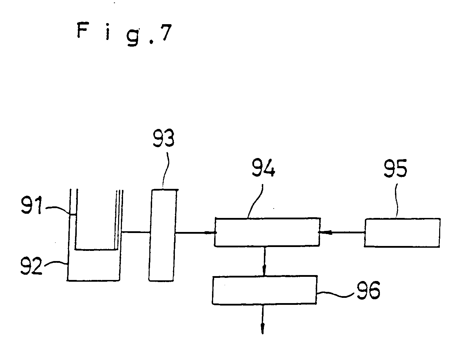

In order to detect an intensity of chemical luminescence, a cylindrical photometric cell 91 made of glass or plastics has been fixedly mounted on an integrated spherical cell holder 92 and a photomultiplier tube 94 as an optical detector has been provided through a shutter 93 so as to face to said photometric cell 91 in order to detect a quantity of chemical luminescence generated within the photometric cell 91 by the so-called batch-type measuring method, as shown in Fig. 7. In addition, referring to Fig. 7, reference numeral 95 designates a high-voltage power source and reference numeral 96 designates an amplifier.

However, according to the conventional chemical luminescence-detecting apparatus as shown in the above described Fig. 7, merely one photomultiplier tube 94 is provided for the photometric cell 91, so that a range, within which said quantity of chemical luminescence can be measured, is limited in case where the measurement is carried out under the same one condition, and thus it has been necessary to carry out the measurement with regulating the measuring conditions, for example a supply voltage from said high-voltage power source 95, a value of feedback resistance in said amplifier 96 and the like, of the photomultiplier tube 94 by various kinds of devices.

Further, the enzyme immuno assay has been recently carried out on the basis of the chemical luminescence method in the enzyme immuno measurement. However, it was problematically to use the above described conventional chemical luminescence-detecting apparatus as it is. A large number of items has to be randomly measured in the enzyme immuno measurement, so that a range of quantity of light to be measured is remarkably wide and thus the measurement can not be carried out by means of a single optical detector.

So, an apparatus has already been developed for detecting a chemical luminescence in a photometric cell by means of a plurality of optical detectors different in sensitivity of measurement.

However, in case where said apparatus for detecting a chemical luminescence is calibrated by means of, for example, two optical detectors different in sensitivity of measurement, a calibration curve for each optical detector is obtained but outputs from both optical detectors are different in level, so that merely a discontinuous calibration curve can be obtained. However, in order to carry out the conversion into concentration over a wide range, one piece of continuous calibration curve is necessary and therefore the calibration at discontinuous points is necessary.

A spectro photofluorometer known from JP-A-59 125043 comprises an exiting spectrometer, a sample cell, and a single photo detector for detecting light generated by fluorescence in the sample cell. The output of the photo detector is supplied to a CPU which is connected with a memory. The photo detector of the spectro photofluorometer can be operated in a high sensitivity mode and in a low sensitivity mode.

In order to determine a sensitivity ratio the intensity of a standard light source is measured by the photo detector in the high sensitivity mode and in the low sensitivity mode and the sensitivity ratio $a = Ih / Il$ is calculated from the outputs $Ih$ and $Il$ of the photo detector in the high sensitivity mode and in the low sensitivity mode, respectively. Further, zero set values a, b are determined for the high and low sensitivity mode by use of an blank sample, i. e. by a sample containing only the pure solvent.

Thus, the calibration is carried out by using a blank sample and a standard light source and not by using standard samples having a known concentration.

## SUMMARY OF THE INVENTION

The Object of the present invention is to provide a calibrating method for calibrating an enzyme immuno assay system in a simplified manner in order to obtain a continuous calibration curve and to conduct measurements over a wide range of concentrations.

This Object is achieved by the calibration method of claim 1.

According to the method of the present invention, one piece of continuous calibration curve can be obtained over a wide range of concentration, so that the apparatus can be calibrated so that also the respective items contained in a blood in various concentrations can be measured under the same one conditions.

## BRIEF DESCRIPTION OF THE DRAWINGS

Preferred embodiments of the present invention and a conventional apparatus are shown in the Figures, in which

Fig. 1    is a sectional view showing one example of a chemical luminescence-detecting apparatus according to the present invention;

Fig. 2    is a block diagram roughly showing a construction of circuit of said apparatus shown in Fig. 1;

Fig. 3    is a schematic diagram showing a relation among an output from a high sensitivity photomultiplier tube, an output from a low sensitivity photomultiplier tube and a concentration of luminous substance;

Fig. 4    is a whole perspective view showing an inside of an enzyme immuno assay system with the chemical luminescence-detecting apparatus incorporated;

Fig. 5    is a partially opened main block side view of said system;

Fig. 6    is a plan view showing main components of said system; and

Fig. 7    is a block diagram showing a conventional chemical luminescence-detecting apparatus.

## DESCRIPTION OF THE PREFERRED EMBODIMENT

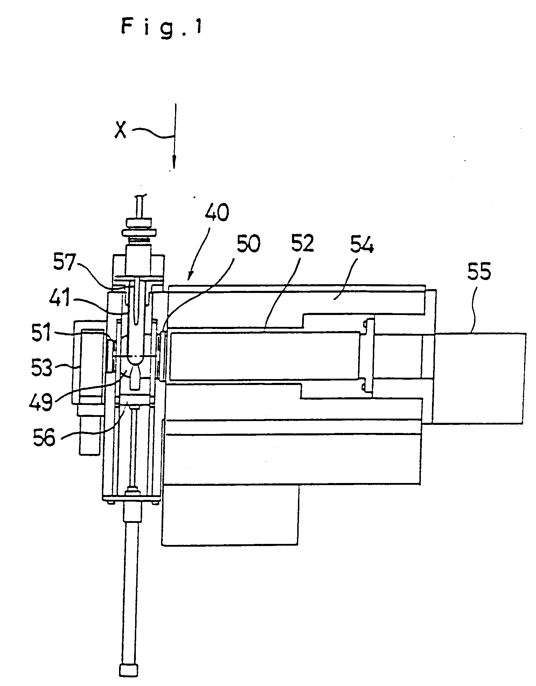

The preferred embodiments of the present invention will be described below with reference to the drawings. Figs. 1 to 6 show one preferred embodiment of the present invention.

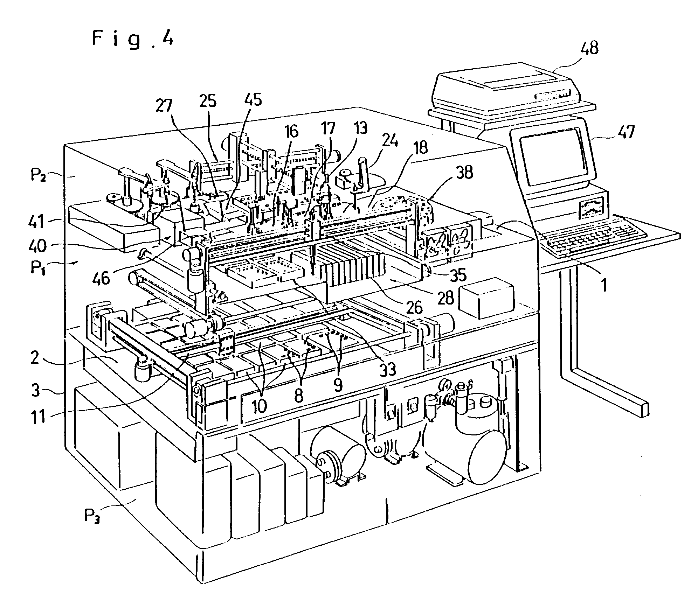

At first, before the chemical luminescence-detecting apparatus according to the present invention is described, the construction of the enzyme immuno assay system with the chemical luminescence-detecting apparatus incorporated therein is described with reference to Figs. 4 to 6.

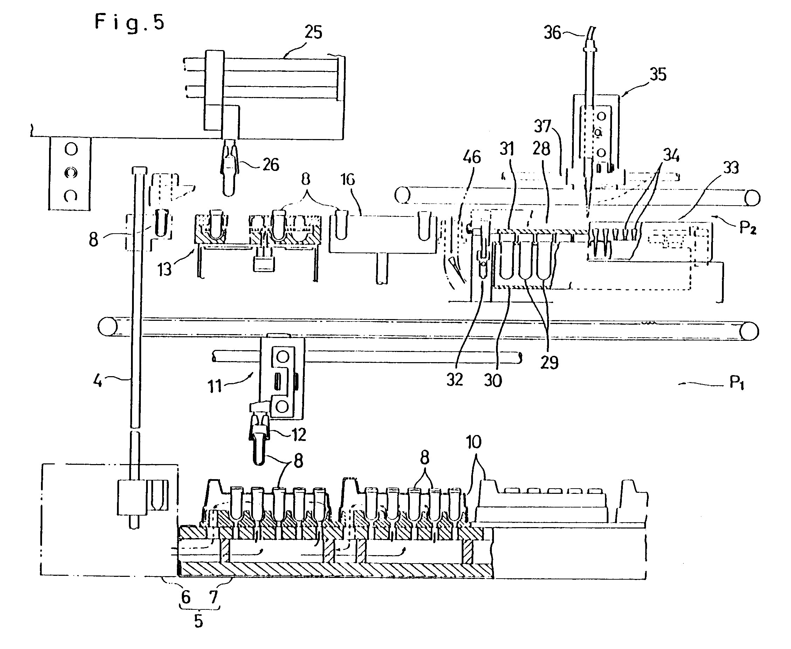

Referring to Fig. 4, reference numerals 1, 2 designate partition plates dividing an inner space of an apparatus case 3 into three spaces $P_1$, $P_2$, $P_3$, whereas $P_2$ is an upper space, $P_1$ a central space and $P_3$ a lower space. As shown in Fig. 4, a tube conveying elevator 4 is provided ranging from the central space $P_1$ to the upper space $P_2$. Further, reference numeral 5 in Fig. 5 designates an antibody immobilized tube cooling device comprising a suction-exhaust portion 6 communicatedly connected with a cooler (not shown) provided in the lower space $P_3$ and a cooling case 7 communicatedly connected with said suction-exhaust portion 6. Said cooling case 7 can be freely drawn out through the front side of said apparatus case 3.

Referring again to Fig. 4, reference numeral 8 designates an antibody immobilized tube with an antibody immobilized on an inner surface of a bottom thereof, and an upper opening thereof being sealed with an aluminum foil, whereas reference numeral 9 designates a dilution tube. Said tubes 8, 9 are supported by a tube-supporting case 10 having its lower side opened, said case 10 being detachably placed on an upper surface portion of said cooling case 7 so as to form a cooling duct.

Reference numeral 11 designates a tube-conveying mechanism to be moved horizontally in two-dimensional directions, which is provided with a freely elevatable vessel chuck 12 (refer to Fig. 5) for conveying the antibody immobilized tube 8 (the dilution tube 9, if necessary) to a conveyance-beginning end of said elevator 4.

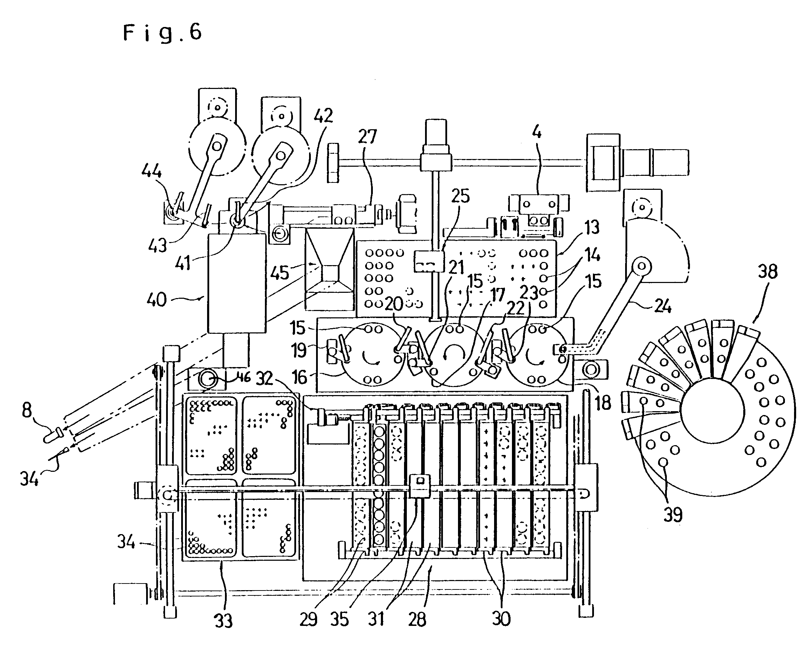

As shown in Fig. 6, reference numeral 13 designates a constant temperature shaker provided with a plurality of tube holding portions 14 and first to third rotors 16 to 18, each having a plurality of holding holes 15 for receiving an antibody immobilized tube 8. Said rotors 16, 17, 18 are arranged on the front side of said constant temperature shaker 13. A washer 19 and a diluent dispenser 20 are arranged around said first rotor 16, a washer 21 and a substrate solution dispenser 22 around said second rotor 17, and a washer 23 and an enzyme conjugated antibody reagent dispenser 24 around said third rotor 18 so that they may be rotated by an appointed angle in the direction shown by, for example, an arrow in the drawing.

3

Reference numeral 25 designates a tube-conveying mechanism provided with a tube chuck 26 (refer to Fig. 5) freely movable in three-dimensional directions for conveying the antibody immobilized tube 8 conveyed by means of the elevator 4 in an area ranging from the constant temperature shaker 13 to a sample portion 27 through the first to third rotors 16 to 18.

Reference numeral 28 designates a sample tube-housing portion in which sample tube-housing cases 30, each housing a plurality of sample tubes 29 with a sample (for example serum) poured therein in a row, stand in a line in the right and left direction (refer to Figs. 5, 6). And, referring to Figs. 5 and 6, reference numeral 31 designates a cover member for individually closing upper openings of said sample tube-housing cases 30. A cover member-closing mechanism 32 is provided on one end side in the direction, in which said tubes 29 stand in a row.

Reference numeral 33 designates a stock portion of a pipette tip 34. Reference numeral 35 designates a sample dispenser mechanism to be moved horizontally in two-dimensional directions. Said sample dispenser mechanism 35 is provided with a freely elevatable probe 37 communicatedly connected with a suction-exhaust pipe 36 at an upper portion thereof and said pipette tip 34 at a lower end thereof. By a descending movement of said probe 37 within said stock portion 33 a sample may be sucked into the pipette tip 34 from the sample tube 29 by a sucking action and said sample may be discharged in the antibody immobilized tube 8 held in the first rotor 16 by an exhausting action (refer to Fig. 5).

Reference numeral 38 designates a stock portion of reagent bottles 39 with said enzyme conjugated antibody reagent poured (refer to Fig. 6).

Referring to Fig. 4 and Fig. 6, reference numeral 40 designates a photometric portion provided with a photometric cell 41 formed of a glass tube, reference numeral 42 designates a reactant dispenser for pouring a reactant contained in the antibody immobilized tube 8 conveyed to said sample portion 27 into said photometric cell 41, reference numeral 43 designates a reagent dispenser for pouring a luminescent reagent (for example luminol solution) into the photometric cell 41, and reference numeral 44 designates a washer for the photometric cell 41. Reference numeral 45 designates a recovery portion for the antibody immobilized tube 8 and reference numeral 46 designates a discharging portion for the pipette tip 34.

With the enzyme immuno assay system having the above described construction, the enzyme immuno assay by, for example, the two-step sandwich method is carried out as follows:

The antibody immobilized tube 8 with the antibody corresponding to the item to be measured immobilized thereon is taken into said tube-holding hole 15 of the first rotor 16 by means of said tube-conveying mechanism 11 on the lower side, the elevator 4 and said tube-conveying mechanism 25 on the upper side. The aluminum foil sealing the upper opening of the antibody immobilized tube 8 is broken in the midway of said process of taking out the tube 8.

On the other hand, the probe 37 is provided with the pipette tip 34 at a lower end thereof so that the sample may be sucked into the pipette tip 34 from the sample tube 29 and then poured into the antibody immobilized tube 8 positioned in the first rotor 16. After that, the pipette tip 34 is discharged via said discharging portion 46.

Upon rotating the first rotor 16 by said appointed angle, a diluent is poured into the antibody immobilized tube 8, into which the sample has been poured, and then the antibody immobilized tube 8 is set into the constant temperature shaker 13 followed by shaking for an appointed time at a constant temperature of about the body heat to carry out a first immuno reaction.

The antibody immobilized tube 8 is taken out into the second rotor 17 to be washed and then subjected to a so-called B/F separation followed by pouring an appointed dose of enzyme conjugated antibody reagent corresponding to said item to be measured and setting into the constant temperature shaker 13 again to carry out a second immuno reaction.

Subsequently, the antibody immobilized tube 8 is taken out into the rotor 18 to be washed and then an appointed quantity of substrate solution is poured into the antibody immobilized tube 8 followed by setting into the constant temperature shaker 13 again to carry out an enzyme reaction for an appointed time. Hydrogen peroxide is generated in the antibody immobilized tube 8 in a quantity corresponding to a quantity of substance to be measured by this reaction.

After the enzyme reaction, the antibody immobilized tube 8 is conveyed to the sample portion 27 where the reaction solution containing hydrogen peroxide is added in the photometric cell 41, into which said luminescent reagent has been previously poured, to carry out a luminescent reaction. On the other hand, the antibody immobilized tube 8 is thrown into the discharging portion 45.

A luminescent quantity during the above described luminescent reaction is electrically measured and operated by means of a computer to display an analytical result (concentration of luminescent substance) on a monitor 37. Said result is recorded by means of a printer 48.

In said photometric portion 40 of the above described enzyme immuno assay system as shown in Fig. 1,

4

the photometric cell 41 is held by a cell holder 49 having an integrated spherical shape. A high sensitivity photomultiplier tube (hereinafter referred to as HPMT) 52 and a low sensitivity photomultiplier tube (hereinafter referred to as LPMT) 53 are disposed through interference filters 50, 51, respectively, on both sides in the right and left direction of the photometric cell 41 so that they may stand on one straight line as observed in the direction of an arrow X. Reference numeral 54 designates a housing for said HPMT 52, and said housing 54 is provided with a cooler (not shown) for reducing the dark current of the HPMT 52. In addition, reference numeral 55 designates an amplifier of the HPMT 52, reference numeral 56 designates a shutter, and reference numeral 57 designates a reactant-pouring nozzle.

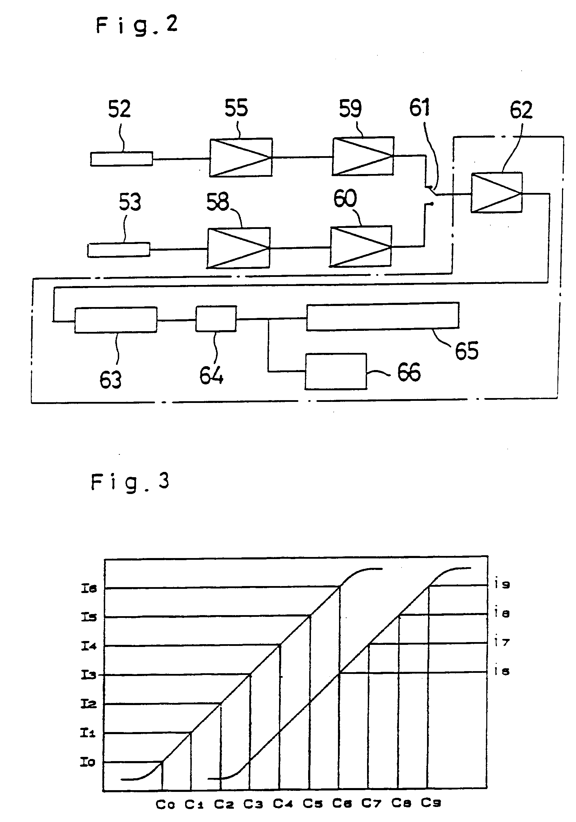

In case where the PMTs 52, 53 different in sensitivity are used to detect the chemical luminescent quantity, as described above, a signal from the PMT 52, however, is greatly different from that of the PMT 53 in level, so that a concentration signal must be obtained by converting said signals into a signal quantity, separately. In order to achieve this, the apparatus according to the present invention has a construction as shown in Fig. 2.

That is to say, Fig. 2 shows a connecting relation of the HPMT 52 with the LPMT 53. Referring to Fig. 2, reference numerals 55, 58 designate amplifiers, reference numerals 59, 60 designate log amplifiers, reference numeral 61 designates a changeover switch, reference numeral 62 designates an A/D converter, reference numeral 63 designates an inverse log converter, reference numeral 64 designates an integrator, and reference numeral 65 designates a display and reference numeral 66 designates a memory.

In addition, said log amplifiers 59, 60 and said inverse log converter 63 are not always required depending upon a measuring range and said A/D converter 62. Furthermore, the position of the changeover switch 61 is not limited to the position shown. That is to say, the changeover switch 61 may also be disposed at the output sides of two A/D converters (for use in the HPMT and the LPMT) or at the output sides of two integrators. In Fig. 2 the changeover switch 61 is connected to the input portion of said A/D converter 62.

The changeover switch 61 is an analog switch for alternately putting the output signal from the HPMT 52 and the output signal from the LPMT 53 in the A/D converter 62 every 50 msec to put the data in two.

In this preferred embodiment, the radiant life is usually about 10 seconds, and, as described above, the output from the detector is alternately taken out one by one every 50 msec, so that, after all, the output from the respective detectors is divided into 200 pieces to be put into the computer. (The integral value of the respective outputs becomes the datum adopted in the operation of concentration).

The signal from the HPMT 52 and the signal from the LPMT 53, which have been analogized in the A/D converter 62, are subjected to the inverse analog operation in the computer to be memorized. In this time, the signal from the HPMT 52 is preferentially adopted as the datum for the operation of concentration, and, in the case where the signal from the HPMT 52 exceeds the regulation current, the signal from the LPMT 53 is adopted and then the output from the LPMT 53 is multiplied by a factor determined by a ratio of the output from the HPMT 52 to said output from the LPMT 53 based on previously determined luminescent intensities.

That is to say, referring to Fig. 3 showing a relation among the output from the HPMT 52, the output from the LPMT 53 and a concentration of luminescent substance, $C_0$ to $C_9$ on an axis of abscissa designate known concentrations of said luminescent substance, $I_0$ to $I_6$ on an axis of ordinate on the left side designate the output from the HPMT 52, and $i_6$ to $i_9$ on an axis of ordinate on the right side designate the output from the LPMT 53. Accordingly, said ratio of the output from the HPMT 52 to the output from the LPMT 53 based on the luminescent intensities can be determined by the use of this relation.

Now, provided that the output from the HPMT 52 is $I_r$ and the output from the LPMT 53 is $i_r$, the ratio can be determined as $I_r/i_r$ (= A).

Thus, the ratio of the output from the HPMT 52 to the output from the LPMT 53 is determined. If the output from the HPMT 52 exceeds the regulation current during the measurement, it is detected by means of the LPMT 53 and an output i from the LPMT 53 converted into the output from the HPMT 52 (I) can be expressed by I = i x A.

The standard serums having known concentrations for preparing the calibration curve and a regression (method of establishing the new calibration curve) will be described below with reference to Fig. 3.

(1) In the case where the concentration of the standard serum is $C_0$ to $C_2$, the calibration curve is prepared by the use of data $I_0$ to $I_2$ of the HPMT 52. But, in the case where the output of the HPMT 52 exceeds the regulation value of current, the conversion value of output of the LPMT 53 is determined by the use of the output ratio, which has been previously determined before the measurement, and the regression is carried out by the use of the outputs of the HPMT 52 at the respective concentrations and the conversion values of output to prepare the calibration curve, whereby calculating the concentration from the calibration curve.

(2) In the case where the concentration of the standard serum is $C_0$ to $C_6$, the calibration curve is prepared by the use of data $I_0$ to $I_6$ of the HPMT 52. But, in the case where the output of the HPMT 52 exceeds the regulation value of current, the conversion value of output of the LPMT 53 is determined by the use of the

output ratio $I_6/i_6$ determined at $C_6$, and the calibration curve is prepared in the same manner as in the above described (1) to calculate the concentration from this calibration curve.

(3) In the case where the concentration of the standard serum is $C_2$ to $C_7$, the calibration is prepared by converting the signal $i_7$ of the output of the LPMT 53 exceeding the regulation by the use of the output ratio $I_6/i_6$ determined at $C_6$ to determine the conversion value of output of the HPMT 52 and the calibration curve is prepared in the same manner as in the above described (1) to calculate the concentration from this calibration curve.

(4) In the case where the concentration of the standard serum is $C_6$ to $C_9$, the calibration curve is prepared by the use of data $i_6$ to $i_9$ of the LPMT 53. But, in the case where the output of the LPMT 53 is below the regulation value of current, the regression is carried out by the use of the outputs of the HPMT 52 at the respective concentrations and the conversion values of output to prepare the calibration curve, whereby calculating the concentration from this calibration curve. The conversion values of output of the LPMT 53 are determined by the use of the output ratios which have been previously determined before the measurement.

(5) In the case where the concentration of the standard serum is $C_7$ to $C_9$, the calibration curve is prepared by the use of data $i_7$ to $i_9$ of the LPMT 53. But, in the case where the output of the LPMT 53 is below the regulation value of current, the conversion value of output of the LPMT 53 is determined by the use of the output ratio, which has been previously determined before the measurement, and the regression is carried out by the use of the outputs of the LPMT 53 at the respective concentrations and the conversion values of output to prepare the calibration curve, whereby calculating the concentration from the calibration curve.

As described above, the methods of preparing the calibration curve are different depending upon the concentration of the standard serum but one piece of continuous calibration curve can be obtained in every case.

The equation for calculating the concentration can be expressed by the following equation from the output of the HPMT 52 and the conversion value of the output of the LPMT 53 converted into the output of the HPMT 52:

$$C = f(I)$$

But, in the calibration at one point, the new coefficient $A'$ is determined by the following equation:

$$I_r'/i_r' = A'$$

and if the conversion value $I'$ obtained by reconverting the preceding output i of the LPMT 53 by the coefficient $A'$ is expressed by the following equation:

$$I' = i \times A'$$

the new equation for calculating the concentration is expressed by the following equation:

$$C = f(I')$$

As described above, according to the present invention, one piece of continuous calibration curve can be obtained over a wide range of concentration, so that the apparatus can be calibrated so that also the respective items contained in a blood in various concentrations can be measured under the same conditions.

## Claims

1. A method for calibrating an enzyme immuno assay system adapted to detect an intensity of a chemical luminescence generated in a photometric cell (41) by means of two optical detectors (52, 53) different in sensitivity of measurement,

   **characterized in that**

   a calibration curve is prepared by the use of standard samples having known concentrations to be memorized,

   one of said standard samples is measured by said two optical detectors (52, 53) to determine a coefficient by dividing the output of one of said two detectors (52; 53) through the output of the other detector (53; 52),

   conversion values of outputs from one of said two optical detectors (52, 53) are determined by multiplying the outputs with said coefficient, and

   said calibration curve is corrected on the basis of said conversion values of outputs to establish one piece of new continuous calibration curve.

2. The method as claimed in claim 1, **characterized in that** conversion values $(I')$ for the high sensitivity detector (52) are determined by multiplying the outputs $(i)$ of the low sensitivity detector (53) with a coefficient $(A')$ calculated at one point $(r)$ of the calibration curve by dividing the output $(I_r')$ of the high sensitivity detector (52) through the output $(i_r')$ of the low sensitivity detector (53).

3. The method as claimed in claim 1, **characterized in that** conversion values for the low sensitivity detector (53) are determined by multiplying the outputs of the high sensitivity detector (52) with a coefficient calculated at one point of the calibrating curve by dividing the output of the low sensitivity detector (53) through the output of the high sensitivity detector (52).

**Patentansprüche**

1. Verfahren zum Kalibrieren eines Systems für Enzym-Immuntests, das so ausgebildet ist, daß es die Intensität von in einer Photometriezelle (41) erzeugter Chemolumineszenz mittels zweier optischer Detektoren (52, 53) mit verschiedenen Meßempfindlichkeiten mißt;
   **dadurch gekennzeichnet, daß**
   - eine abzuspeichernde Kalibrierkurve unter Verwendung von Standardproben mit bekannten Konzentrationen erstellt wird;
   - eine der Standardproben mittels der zwei optischen Detektoren (52, 53) gemessen wird, um einen Koeffizient dadurch zu bestimmen, daß das Ausgangssignal eines der zwei Detektoren (52; 53) durch das Ausgangssignal des anderen Detektors (53; 52) geteilt wird, wobei Umsetzungswerte für die Ausgangssignale eines der zwei optischen Detektoren (52, 53) dadurch bestimmt werden, daß die Ausgangssignale mit dem Koeffizient multipliziert werden; und
   - die Kalibrierkurve auf Grundlage der Umsetzwerte für die Ausgangssignale korrigiert wird, um ein Stück einer neuen kontinuierlichen Kalibrierkurve zu erstellen.

2. Verfahren nach Anspruch 1, **dadurch gekennzeichnet, daß** Umsetzwerte (I') für den Detektor hoher Empfindlichkeit (52) dadurch bestimmt werden, daß die Ausgangssignale (i) des Detektors niedriger Empfindlichkeit (53) mit einem Koeffizient (A') multipliziert werden, der an einem Punkt (r) der Kalibrierkurve dadurch berechnet wurde, daß das Ausgangssignal ($I_r'$) des Detektors mit hoher Empfindlichkeit (52) durch das Ausgangssignal ($i_r'$) des Detektors mit niedriger Intensität (53) geteilt wurde.

3. Verfahren nach Anspruch 1, **dadurch gekennzeichnet, daß** Umsetzwerte für den Detektor niedriger Empfindlichkeit (53) dadurch bestimmt werden, daß die Ausgangssignale des Detektors (52) mit hoher Empfindlichkeit mit einem Koeffizient multipliziert werden, der an einem Punkt der Kalibrierkurve dadurch berechnet wurde, daß das Ausgangssignal des Detektors mit niedriger Empfindlichkeit (53) durch das Ausgangssignal des Detektors mit hoher Empfindlichkeit (52) geteilt wurde.

**Revendications**

1. Un procédé d'étalonnage d'un système de test immunologique, effectué sur des enzymes, apte à détecter l'intensité de la luminescence chimique générée dans une cellule photométrique (41) au moyen de deux capteurs optiques (52, 53) présentant des sensibilités de mesure différentes, caractérisé
   en ce que l'on prépare une courbe d'étalonnage à partir d'échantillons standards présentant des concentrations connues à mémoriser,
   en ce que l'un desdits échantillons standards est mesuré par lesdits deux capteurs optiques (52, 53) pour déterminer un coefficient obtenu par division du signal de sortie de l'un desdits deux capteurs (52; 53) par le signal de sortie de l'autre capteur (53; 52),
   en ce que l'on détermine les valeurs de conversion des signaux de sortie de l'un desdits deux capteurs optiques (52, 53), en multipliant les signaux de sortie par ledit coefficient,
   et en ce que l'on corrige ladite courbe d'étalonnage sur la base desdits valeurs de conversion des signaux de sortie pour établir un exemplaire de la nouvelle courbe d'étalonnage continue.

2. Le procédé selon la revendication 1, caractérisé en ce que l'on détermine les valeurs de conversion (I') pour le détecteur à grande sensibilité (52), en multipliant les signaux de sortie (i) du détecteur à faible sensibilité (53) par un coefficient (A') calculé en un point (r) de la courbe d'étalonnage par division du signal de sortie ($I_r'$) du capteur à sensibilité élevée (52) par le signal de sortie ($i_r'$) du capteur à faible sensibilité (53).

3. Le procédé selon la revendication 1, caractérisé en ce que l'on détermine les valeurs de conversion pour le capteur à faible sensibilité (53) en multipliant les signaux de sortie du capteur à sensibilité élevée (52) par un coefficient calculé en un point de la courbe d'étalonnage par division du signal de sortie du capteur à faible sensibilité (53) par le signal de sortie du capteur à sensibilité élevée (52).

Fig.1

Fig.2

Fig.3

Fig.4

EP 0 446 858 B1

Fig.5

Fig.6

Fig.7